# FASCICULE DE BREVET EUROPEEN

(11) **EP 3 047 283 B1**
(45) Date de publication et mention de la délivrance du brevet: **18.04.2018**
(21) Numéro de dépôt: 14784295.9
(22) Date de dépôt: 16.09.2014
(51) Int. Cl.: G01N 33/82, C11D 7/26, C11D 7/50, C23G 5/028

(54) **SOLUTION DE DISSOCIATION DE LA VITAMINE D DE SA PROTÉINE DE LIAISON, PROCÉDÉ DE DÉTECTION ASSOCIÉ ET UTILISATION**
LÖSUNG ZUM ABTRENNEN VON VITAMIN D VON EINEM VITAMIN-D BINDENDEN PROTEIN, ENTSPRECHENDES DETEKTIONSVERFAHREN UND VERWENDUNG DAVON
SOLUTION FOR DISSOCIATING VITAMIN D FROM VITAMIN D-BINDING PROTEIN, ASSOCIATED DETECTION METHOD AND USE

(30) Priorité: 17.09.2013 FR 1358940
(43) Date de publication de la demande: 27.07.2016
(73) Titulaire: Biomérieux, 69280 Marcy l'Étoile (FR)
(72) Inventeur: MEUNIER, Valérie, F-69890 La Tour de Salvagny (FR); MOREAU, Emmanuel, F-69890 La Tour de Salvagny (FR)
(74) Mandataire: Sarlin, Laure V.
(86) Numéro de dépôt international: PCT/FR2014/052296
(87) Numéro de publication internationale: WO 2015/040319

(56) Documents cités:
- WO-A1-2007/039194
- WO-A1-2012/091569
- WO-A1-2012/136720
- GB-A- 2 166 153
- US-A- 5 648 325
- US-A1- 2005 209 468
- MARY BEDNER ET AL: "Development and comparison of three liquid chromatographyatmospheric pressure chemical ionization/mass spectrometry methods for determining vitamin D metabolites in human serum", JOURNAL OF CHROMATOGRAPHY, ELSEVIER SCIENCE PUBLISHERS B.V, NL, vol. 1240, 28 mars 2012 (2012-03-28), pages 132-139, XP028482021, ISSN: 0021-9673, DOI: 10.1016/J.CHROMA.2012.03.091 [extrait le 2012-04-05]

## Description

La présente invention concerne le domaine technique de la détection de la vitamine D. Plus précisément, l'invention a pour objet l'utilisation d'une association d'un surfactant fluoroalkyle et d'un alcool pour libérer la vitamine D et/ou un de ses métabolites de la protéine de liaison de la vitamine D, une solution contenant une telle association, un procédé de détection/quantification *in vitro* de la vitamine D et/ou d'au moins un métabolite de la vitamine D utilisant une telle association, ainsi qu'un kit de détection/quantification par immunodosage mettant en oeuvre une telle association.

La vitamine D est une substance importante ayant de nombreuses implications dans les processus biologiques du corps humain et animal. La vitamine D, biologiquement active, est connue pour réguler entre autre la fixation du calcium à partir de l'intestin, la minéralisation osseuse, et influence un grand nombre d'autres voies métaboliques comme par exemple le système insulinaire. Une déficience ou un excès en vitamine D peut avoir diverses conséquences. En particulier, il est connu qu'une déficience en vitamine D entraîne des maladies sévères telles que l'ostéoporose et le rachitisme.

Par ailleurs, un excès de vitamine D, notamment dû à un surdosage est toxique. En particulier, un taux important de vitamine D peut entraîner une hyper-calcémie causée par une augmentation de l'absorption intestinale du calcium. D'autres effets toxiques de la vitamine D se manifestent par une augmentation de la pression sanguine, des troubles gastro-intestinaux tels que l'anorexie, les nausées, souvent suivies d'une production excessive d'urine, une polydipsie, de la fatigue, de la nervosité, un prurit ou encore une insuffisance rénale.

Comme mentionné dans la demande WO 2012/091569, il a été également mis en évidence, ces dernières années, que la vitamine D modulait la fonction immunitaire et réduisait l'inflammation. Il a été également suggéré que la vitamine D pouvait prévenir les cancers du côlon, des ovaires et du sein.

Par conséquent, il est important de pouvoir doser ou quantifier, la vitamine D, c'est-à-dire en déterminer sa concentration, de manière à mettre en évidence des éventuelles carences ou excès.

La vitamine D est présente dans l'organisme sous deux formes, à savoir la vitamine D₂ (ergocalciférol) et la vitamine D₃ (cholécalciférol) dont les formules (dans lesquelles les positions de la vitamine D sont indiquées conformément à la nomenclature des stéroïdes) sont reprises ci-après.

### vitamine D₂ (ergocafciférol) :

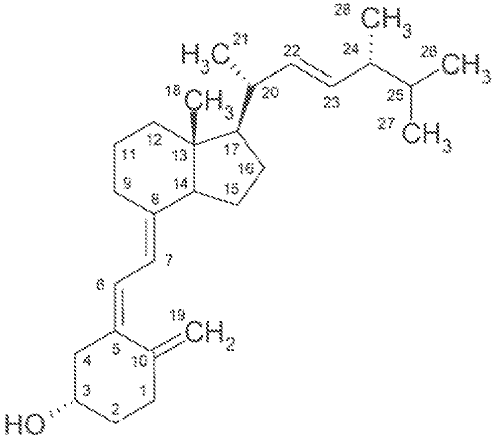

### vitamine D₃ (cholécaldférol) :

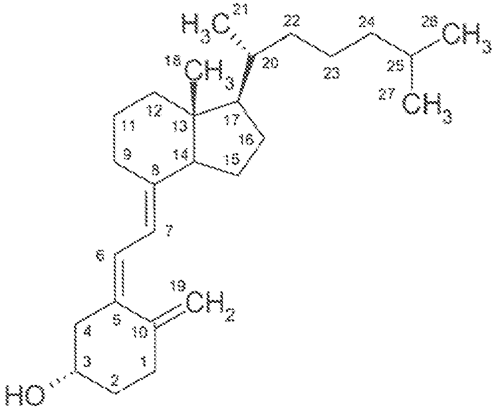

La vitamine D₂ est la forme exogène de la vitamine D, provenant de la nourriture. La vitamine D₃ est la forme endogène de la vitamine D produite par l'organisme sous l'action sur la peau des rayons ultraviolets de la lumière solaire. La vitamine D₃ qui est produite au niveau de la peau, se lie à la protéine de liaison de la vitamine D qui la transporte dans le foie. Les deux formes peuvent provenir de suppléments nutritionnels. Différents métabolites de la vitamine D sont produits. En particulier, une métabolisation en deux étapes a lieu, la première étape consistant en la production de 25-hydroxyvitamine D (D₂ ou D₃), suivie par la production de 1,25 dihydroxyvitamine D (D₂ ou D₃).

La mesure du taux de vitamine D elle-même présente un intérêt limité, étant donné que sa concentration fluctue fortement en fonction de l'alimentation. Il en va de même des métabolites 1,25-dihydroxyvitamine D qui sont présents en concentration assez faible et qui fluctuent également.

Les formes circulantes de la vitamine D sont essentiellement les métabolites du type 25-hydroxyvitamine D. Aussi, le dosage de la 25-hydroxyvitamine D est le mode privilégié pour obtenir des informations sur la concentration globale de vitamine D chez un patient.

La liaison de la 25-hydroxyvitamine D, ou plus généralement de la vitamine D et de ses métabolites, à la protéine de liaison de la vitamine D (« D Binding Protein » ou DBP) complique le dosage de ces composés. Pour obtenir un dosage adéquat, il est nécessaire de libérer l'haptène à doser de la DBP, en obtenant leur dissociation. Aussi, différentes solutions ont été proposées pour obtenir la libération de la vitamine D et de ses métabolites, après dissociation de la DBP, avant détection de ces derniers.

Différentes techniques listées dans la demande de brevet WO 2007/039194 et dans la demande de brevet WO 2012/091569 ont été développées. Seules quelques-unes seront listées ici.

Une technique ancienne, notamment utilisée dans la demande WO 99/67211, consiste à préparer un échantillon de plasma ou de sérum pour la détermination de la vitamine D par précipitation à l'éthanol. Les précipités sont ensuite éliminés, pour récupérer le surnageant éthanolique contenant les métabolites solubles de la vitamine D. La précipitation par un alcool ou un autre solvant organique tel que l'acétonitrile a été couramment utilisée par le passé. Cependant, cette technique ne peut être automatisée et nécessite de nombreuses opérations manuelles (ajout de solvants au sérum, mélange, centrifugation, récupération de la phase organique, séchage sur colonne ou autre, re-suspension dans un solvant liquide), ce qui la rend obsolète de nos jours du fait de la très grande variation inter-opérateurs observée.

La demande WO2012/136720, au nom de Immunodiagnostik, décrit un procédé et un kit pour la détermination quantitative de métabolites de la vitamine D dans le sang, avec lesquels une quantité de sang est immobilisée de façon pré-analytique sur un matériau de sorption solide. Pour l'analyse quantitative, la tache de sang séchée sur le matériau de sorption est attaquée au moyen d'un tampon de dissolution aqueux contenant un détergent et les métabolites de la vitamine D sont élués au moyen d'une solution organique protique ayant une permittivité inférieure à 35. Le solvant protique organique utilisé, peut être le méthanol, le butanol ou un mélange méthanol/isopropanol comme décrit au paragraphe [0049].

La demande de brevet WO 2007/039194 propose, quant à elle, d'utiliser une solution contenant de 5 à 30% en volume d'un ou plusieurs réactifs amphiphiles choisis parmi le diméthylsulfoxyde (DMSO) et les amides organiques liquides, et optionnellement de 0,7 à 8 % en volume d'un alcool à chaine courte (en C₁-C₃). Les composés amphiphiles utilisés dans ce document sont des produits toxiques, voire dangereux dans le cas du DMSO.

Les demandes de brevet WO 2011/122948 et WO 2012/091569 au nom de Future Diagnostics décrivent un immunodosage et un agent permettant de libérer la vitamine D de sa protéine de liaison qui utilise un surfactant fluoroalkyle. La Demanderesse qui a utilisé cette solution, a cependant constaté que la dissociation obtenue était encore insuffisante.

L'objectif de l'invention est de proposer une technique de dissociation plus performante, qui soit facile à mettre en oeuvre et qui conduise à une libération efficace de la vitamine D et de ses métabolites, après dissociation
de la protéine de liaison de la vitamine D (DBP), permettant ensuite une détection et quantification adéquate de ces derniers.

Dans le cadre de la présente invention, l'objectif est de fournir une nouvelle solution pour dissocier la vitamine D ou un de ses métabolites, de la protéine de liaison de la vitamine D (DBP), qui soit plus efficace que les techniques proposées dans l'art antérieur par Future Diagnostics dans ses demandes de brevet WO 2011/122948 et WO 2012/091569. En outre, la solution décrite dans la présente invention ne présente pas les problèmes de toxicité des solutions notamment proposées dans la demande de brevet WO 2007/039194.

Dans ce contexte, l'invention concerne l'utilisation d'au moins un surfactant fluoroalkyle, et en particulier perfluoroalkyle, et d'au moins un alcool comportant de 1 à 4 atomes de carbone, pour dissocier, de la protéine de liaison de la vitamine D, la vitamine D et/ou un métabolite de la vitamine D. Une telle utilisation conjointe permet d'augmenter significativement le taux de dissociation de la vitamine D ou d'un de ses métabolites, de la protéine de liaison de la vitamine D, par rapport à une utilisation similaire différant uniquement par l'absence d'alcool. Dans l'utilisation selon l'invention, le surfactant fluoroalkyle, et en particulier perfluoroalkyle, et l'alcool comportant de 1 à 4 atomes de carbone, sont incorporés dans un échantillon liquide comportant la vitamine D et/ou un métabolite de la vitamine D (nommé analyte ou analyte de la vitamine D), associé à la protéine de liaison de la vitamine D. L'analyte à dissocier est donc en contact à la fois avec le surfactant fluoroalkyle et l'alcool qui, utilisés ensemble, vont permettre une dissociation plus importante, par comparaison avec l'utilisation du même surfactant fluoroalkyle seul. L'utilisation conjointe de surfactant fluoroalkyle, et en particulier perfluoroalkyle, et d'alcool comportant de 1 à 4 atomes de carbone, entraîne une dissociation efficace entre, la protéine de liaison de la vitamine D, et la vitamine D et/ou un de ses métabolites.

En particulier, le surfactant fluoroalkyle et l'alcool sont utilisés en quantités telles que le rapport multiplié par 100 de la masse de surfactant, quand ce dernier est un solide, ou du volume de surfactant, quand ce dernier est un liquide, sur le volume d'alcool, appartient à la gamme allant de 10 à 60%, de préférence à la gamme allant de 15 à 40 %, et préférentiellement dans la gamme allant de 15 à 30%. Le caractère solide ou liquide s'apprécie à température ambiante (à 22°C notamment) et à pression atmosphérique (notamment à 1013 hPa).

De manière avantageuse, le surfactant fluoroalkyle est choisi parmi les acides perfluorocarboxyliques, les acides perfluorosulfoniques et leurs sels, et notamment parmi l'acide perfluorohexanoïque, l'acide perfluoroheptanoïque, l'acide perfluorooctanoïque et leurs sels. L'acide perfluorohexanoïque est également appelé acide perfluorocaproïque ou encore acide undécafluorohexanoïque ; son numéro CAS (« chemical abstracts service registration number ») est 307-24-4. L'acide perfluorooctanoïque est également appelé acide perfluorocaprylique ou encore acide pentadécafluorooctanoïque ; son numéro CAS est le 335-67-1. En général, les sels de ces acides perfluoroalkyles sont solides, alors que les acides libres correspondants sont liquides.

l'acide perfluorohexanoïque, éventuellement sous forme de sel, est le surfactant fluoroalkyle préféré, car il présente une meilleure dégradabilité par rapport aux surfactants fluoroalkyles à chaîne plus longue.

De manière préférée, l'alcool utilisé comporte de 1 à 3 atomes de carbone et est choisi parmi le méthanol, l'éthanol, le *n*-propanol et l'*iso*propanol. Le méthanol est l'alcool préféré dans le cadre de l'invention car il permet d'obtenir des meilleures performances en termes de reproductibilité des résultats de dosage mis en oeuvre après dissociation, et: offre un bon compromis au regard de l'amélioration de la dissociation et de la reproductibilité des résultats obtenus.

De manière particulièrement avantageuse, la dissociation sera réalisée avec de l'acide perfluorohexanoïque et du méthanol.

Dans le cadre de l'invention, la dissociation pourra être réalisée en présence d'un surfactant additionnel choisi, notamment, parmi les copolymères blocs à base d'oxyde d'éthylène et d'oxyde de propylène, les polysorbates et les éthers de polyéthylène glycol.

L'invention est, par exemple, mise en oeuvre pour dissocier la protéine de liaison de la vitamine D, de la 25-hydroxyvitamine D, et en particulier de la 25-hydroxyvitamine D₂ et/ou de la 25-hydroxyvitamine D₃.

L'alcool et le surfactant fluoroalkyle peuvent être incorporés séparément à l'échantillon dans lequel on souhaite obtenir la dissociation, ou incorporés en même temps. Dans ce cas, on utilisera une solution unique, nommée solution de dissociation, afin de minimiser les manipulations.

L'invention a également pour objet de telles solutions comprenant au moins un surfactant fluoroalkyle, et en particulier perfluoroalkyle, et au moins un alcool comportant de 1 à 4 atomes de carbone.

De telles solutions peuvent être qualifiées d'aqueuses et comporteront une part importante d'eau, représentant en général plus de 80% en volume, par rapport au volume total de la solution.

De manière avantageuse, une telle solution comprendra un pourcentage de surfactant fluoroalkyle, exprimé en volume lorsque ce dernier est liquide, ou en masse lorsque ce dernier est solide, par rapport au volume total de la solution, de 0,1 à 3%, de préférence de 1 à 2%. De manière également préférée, une telle solution comprendra un pourcentage en volume d'alcool, par rapport au volume total de la solution, de 0,5 à 10%, de préférence de 2 à 7%.

Les mêmes préférences, quant au choix de l'alcool et du surfactant fluorolakyle et à leur quantité relative, données en relation avec l'utilisation, s'appliquent aux solutions de dissociation selon l'invention.

En particulier, la solution contient le méthanol, en tant qu' alcool comportant de 1 à 4 atomes de carbone et au moins un surfactant fluoroalkyle choisi parmi les acides perfluorocarboxyliques, les acides perfluorosulfoniques et leurs sels, le surfactant fluoroalkyle étant, de préférence, l'acide perfluorohexanoïque de la revendication 11 antérieure.

Selon une variante avantageuse, les solutions de dissociation selon l'invention contiennent, en outre, un autre surfactant choisi parmi les copolymères blocs à base d'oxyde d'éthylène et d'oxyde de propylène, les polysorbates et les éthers de polyéthylène glycol. Sans vouloir être lié par une quelconque interprétation des résultats, un tel surfactant additionnel pourrait améliorer la solubilité de la vitamine D ou de ses métabolites à doser. L'utilisation d'un tel surfactant additionnel, tel que le Pluronic® F-127, qui est un polyol correspondant à un copolymère bloc à base d'oxyde d'éthylène et d'oxyde de propylène, permet notamment d'améliorer la reproductibilité des résultats. Il en ressort que le dosage final est plus fiable. A titre d'exemple non limitatif, on pourra utiliser le Pluronic® F-127 avec une concentration dans la solution de dissociation, qui se situe, de préférence, entre 0,1% et 3% (en volume/volume final de la solution de dissociation).

En général, les solutions de dissociation selon l'invention seront tamponnées, notamment à un pH appartenant à la gamme allant de 6 à 8.

Les tampons classiquement utilisés dans le domaine du diagnostic, pourront être incorporés dans la solution, de manière à obtenir et stabiliser le pH dans la gamme souhaitée. A titre d'exemple, un tampon PBS (tampon phosphate salin) ou tris (tris-hydroxyméthylaminométhane) pourra être utilisé.

Une base pourra être incorporée pour ajuster le pH. Une telle base peut être toute base classiquement utilisée à cette fin, telle que KOH, NaOH, LiOH ou Na₂HPO₄.

Une telle solution de dissociation peut être préparée en mélangeant un tampon dont la concentration se situe, de préférence, entre 1 et 500 mM, un surfactant fluoroalkyle dont la concentration se situe, de préférence, entre 0,1% et 3% (en masse ou en volume en fonction du caractère solide ou liquide du surfactant/volume final de la solution de dissociation) et un alcool dont la concentration se situe, de préférence, entre 0,5% et 10% (volume/volume final de la solution de dissociation) dans de l'eau déminéralisée. Le pH de la solution de dissociation est ajusté selon le tampon choisi, par ajout d'acide ou par ajout de base à une valeur comprise entre 4 et 8, de préférence autour de 7. Quand la solution de dissociation contient un surfactant additionnel, ce dernier peut être introduit à n'importe quel stade.

De manière avantageuse, de telles solutions de dissociation ne contiennent aucun des composés suivants : diméthyl sulfoxyde, diméthylformamide, N,N-diméthylacétamide, tetraméthylurée, N-méthylpyrrolidone, 1,3-diméthyl-3,4,5,6-tétrahydro-2(1H)-pyrimidone, acide triamide hexaméthylphosphorique.

L'invention a également pour objet un procédé de détection et de quantification, *in vitro,* de la vitamine D et/ou d'au moins un métabolite de la vitamine D, dans un échantillon biologique, comprenant les étapes suivantes :
a) une étape de traitement de l'échantillon par incorporation d'au moins un surfactant fluoroalkyle et d'au moins un alcool comportant de 1 à 4 atomes de carbone, de manière à dissocier, la vitamine D et/ou son ou ses métabolite(s), de la protéine de liaison de la vitamine D,
b) une étape de détection et de quantification de la vitamine D et/ou d'au moins un de ses métabolites.

L'étape a) de dissociation sera effectuée avant l'étape b) de détection et de quantification.

De manière avantageuse, dans un tel procédé de détection et de quantification, le traitement de l'étape a) est réalisé par mélange de l'échantillon avec une solution de dissociation selon l'invention. Le plus souvent, de 1 à 20 volumes de solution de dissociation, de préférence de 5 à 10 volumes, de préférence encore de 6 à 8 volumes, et notamment environ 7 volumes de solution, sont utilisés pour 1 volume d'échantillon. Le choix du volume est fonction de la concentration présumée en vitamine D et/ou métabolite de la vitamine D à détecter (nommé analyte à détecter).

De manière avantageuse, le procédé de détection et de quantification selon l'invention sera mis en oeuvre sur un échantillon de sang, de sérum ou de plasma.

Le procédé de détection et de quantification est notamment adapté à la détection et à la quantification de la 25-hydroxyvitamine D₂ et/ou de la 25-hydroxyvitamine D₃.

A l'étape b), la détection et la quantification sont effectuées, de manière préférée, par mise en oeuvre d'un immunodosage, ou encore par spectrométrie de masse.

L'invention concerne également une trousse de détection et de quantification, de la vitamine D et/ou d'au moins un métabolite de la vitamine D, par immunodosage, comprenant une solution de dissociation selon l'invention. Une telle trousse comportera, en outre, un partenaire de liaison à la vitamine D ou à un de ses métabolites et/ou une phase solide sur laquelle est lié un haptène analogue à la vitamine D et/ou au(x) métabolite(s) de la vitamine D à détecter, qui est reconnu par l'anticorps marqué.

Dans le cadre de l'invention, les métabolites de la vitamine D englobent tous les composés qui contiennent le squelette de la vitamine D₂ ou le squelette de la vitamine D₃, et notamment :
- la 25-hydroxyvitamine D qui désigne les métabolites de la vitamine D hydroxylés en position 25, c'est-à-dire la 25-hydroxyvitamine D₂ et la 25-hydroxyvitamine D₃ ;
- les formes 1,25 et 24,25-dihydroxyvitamine D qui désignent les métabolites de la vitamine D dihydroxylés, respectivement en positions 1 et 25, et en positions 24 et 25.

Dans les procédés et utilisations selon l'invention, la dissociation peut être effectuée en incorporant dans l'échantillon à analyser le surfactant fluoroalkyle et l'alcool en C₁-C₄, de préférence en C₁-C₃, sélectionnés, ensemble ou séparément Après cette incorporation, aucune séparation n'est nécessaire, la détection pouvant être effectuée directement sur l'échantillon ainsi obtenu. De préférence, pour limiter les manipulations, une solution déjà préparée contenant le surfactant fluoroalkyle et l'alcool en C₁-C₄, de préférence en C₁-C₃, sélectionnés, et en particulier une solution conforme à l'invention, sera incorporée à l'échantillon.

De manière à favoriser la dissociation, un mélange sera opéré sur l'échantillon contenant le surfactant fluoroalkyle, et notamment le surfactant perfluoroalkyle, et l'alcool en C₁-C₄, de préférence en C₁-C₃, sélectionnés. Un tel mélange peut être effectué avec tout dispositif adapté, et notamment au moyen d'un cône de réaction jouant le rôle de pipette, comme dans la technologie VIDAS® de la Demanderesse (VIDAS® Manuel Instrument, 2005, Chapitre 2 Description fonctionnelle, 2-1 à 2-16, bioMérieux France).

La détection de la vitamine D ou d'un de ses métabolites pourra être effectuée selon toute technique connue de l'homme du métier, et notamment, par mise en oeuvre d'un test utilisant un partenaire de liaison de l'analyte à détecter, et en particulier un test immunologique (également nommé immunodosage), ou encore par spectrométrie de masse.

Bien entendu, le préfixe « immuno » dans le terme « immunodosage », par exemple, n'est pas à considérer dans la présente demande comme indiquant strictement que le partenaire de liaison est nécessairement un partenaire d'origine immunologique, tel qu'un anticorps ou un fragment d'anticorps. En effet, comme cela est bien connu de l'homme du métier, ce terme est plus largement utilisé pour désigner des tests et procédés dans lesquels le partenaire de liaison n'est pas uniquement un partenaire d'origine/de nature immunologique mais consiste, par exemple, en un récepteur de l'analyte que l'on souhaite détecter et/ou quantifier, la condition étant que le partenaire de liaison concerné soit capable de se lier à l'analyte recherché, de préférence de manière spécifique. Ainsi, il est connu de parler du dosage ELISA pour des dosages qui utilisent des partenaires de liaison non immunologiques *stricto sensu,* appelés plus largement en anglais « ligand binding assay », que l'on pourrait traduire en langue française par « dosage utilisant la liaison à un ligand », alors que le terme « immuno » est inclus dans l'intitulé *in extenso* correspondant à l'acronyme ELISA. Dans un souci de clarté et d'uniformité, le terme « immuno » est employé dans la présente demande pour désigner toute analyse biologique utilisant au moins un partenaire de liaison adapté pour se lier à l'analyte recherché et détecter et/ou quantifier ce dernier, de préférence de manière spécifique, même quand ledit partenaire de liaison n'est pas de nature ou d'origine immunologique au sens strict.

Le test immunologique sera, de préférence, un dosage par compétition qui est un dosage largement connu de l'homme du métier et utilisé lorsque l'analyte est un haptène. Il consiste à doser l'analyte, ici la vitamine D et/ou au moins un de ses métabolites, dans l'échantillon, en créant une compétition entre l'analyte de l'échantillon et un analogue de cet analyte. La réaction immunologique est alors mise en évidence grâce à la présence d'un traceur.

L'analogue de l'analyte peut être utilisé dans la réaction de compétition sans couplage préalable ou après couplage à un marqueur pour former un conjugué ou traceur.

Un dosage immunologique par compétition nécessite également la mise en oeuvre d'un partenaire de liaison de l'analyte vis-à-vis duquel l'analogue de l'analyte et l'analyte entrent en compétition. Lorsque l'analogue de l'analyte n'est pas couplé à un marqueur (il n'est pas le traceur mais le partenaire de capture), le partenaire de liaison est alors marqué pour constituer le traceur de la réaction. Lorsque l'analogue de l'analyte est couplé à un marqueur (il est alors le traceur), le partenaire de liaison devient alors le partenaire de capture.

Le signal mesuré émis par le traceur est alors inversement proportionnel à la quantité d'analyte de l'échantillon.

Par marqueur, on entend toute molécule contenant un groupement réactif avec le partenaire de capture ou l'analogue de l'analyte, selon le format, directement sans modification chimique, ou après modification chimique pour inclure un tel groupement, laquelle molécule est capable de générer directement ou indirectement un signal détectable. Un tel groupement réactif est notamment une amine primaire. Une liste non limitative de ces marqueurs de détection directe consiste en :
- les enzymes qui produisent un signal détectable, par exemple, par colorimétrie, fluorescence, luminescence, comme la peroxydase de raifort, la phosphatase alcaline, la β-galactosidase, la glucose-6-phosphate déshydrogénase,
- les chromophores comme les composés fluorescents, luminescents, colorants,
- les molécules radioactives comme le ³²P, le ³⁶S ou le ¹²⁵I,
- les molécules fluorescentes telles que les Alexa ou les phycocyanines, et
- les sels électrochimiluminescents tels que des dérivés organo-métalliques à base d'acridinium ou de ruthénium.

Des systèmes indirects de détection peuvent aussi être utilisés, comme, par exemple, des ligands capables de réagir avec un anti-ligand. Le ligand correspond alors au marqueur pour constituer, avec l'analogue de l'analyte ou le partenaire de liaison, le traceur.

Les couples ligand/anti-ligand sont bien connus de l'homme du métier, ce qui est le cas, par exemple, des couples suivants : biotine/streptavidine, haptène/anticorps, antigène/anticorps, peptide/anticorps, sucre/lectine, polynucléotide/complémentaire du polynucléotide.

L'anti-ligand peut alors être détectable directement par les marqueurs de détection directe décrits précédemment ou être lui-même détectable par un autre couple ligand/anti-ligand, et ainsi de suite.

Ces systèmes indirects de détection peuvent conduire, dans certaines conditions, à une amplification du signal. Cette technique d'amplification du signal est bien connue de l'homme du métier, et l'on pourra se reporter aux demandes de brevet antérieures FR 2781802 ou WO 95/08000 de la Demanderesse.

Selon le type de marquage utilisé, l'homme du métier ajoutera des réactifs permettant la visualisation du marquage ou l'émission d'un signal détectable par tout type d'appareil de mesure approprié, comme, par exemple, un spectrophotomètre, un spectrofluorimètre ou encore une caméra haute définition.

Par partenaire de liaison à la vitamine D ou à l'un de ses métabolites, voire à plusieurs de ces composés, on entend toute molécule capable de se lier à la vitamine D ou à l'un de ses métabolites, voire à plusieurs de ces composés (appelés de façon générale « analyte de vitamine D »). A titre d'exemple de partenaire de liaison de l'analyte de vitamine D, on peut citer les anticorps, les fractions d'anticorps, les nanofitines, les récepteurs à l'analyte de vitamine D, ou toute autre protéine qui est connue pour avoir une interaction avec l'analyte de vitamine D.

Les anticorps partenaires de liaison sont, par exemple, soit des anticorps polyclonaux, soit des anticorps monoclonaux.

Les anticorps polyclonaux peuvent être obtenus par immunisation d'un animal avec, comme immunogène, l'analyte de vitamine D cible, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixé un antigène spécifiquement reconnu par les anticorps, notamment l'immunogène.

Les anticorps monoclonaux peuvent être obtenus par la technique des hybridomes largement connue de l'homme du métier. Les anticorps monoclonaux peuvent être également des anticorps recombinants obtenus par génie génétique, par des techniques bien connues de l'homme du métier.

A titre d'exemple de fragments d'anticorps, on peut citer les fragments Fab, Fab', F(ab')2 ainsi que les chaînes scFv (de l'anglais « Single chain variable fragment »), dsFv (de l'anglais « Double-stranded variable fragment »). Ces fragments fonctionnels peuvent notamment être obtenus par génie génétique.

Les nanofitines (nom commercial) sont de petites protéines qui, comme les anticorps, sont capables de se lier à une cible biologique permettant ainsi de la détecter, de la capturer, ou tout simplement de la cibler au sein d'un organisme.

Les partenaires de liaison utilisés peuvent être spécifiques ou non de l'analyte de vitamine D. Ils sont dits spécifiques, quand ils sont capables de se lier de façon exclusive, ou quasi exclusive, à l'analyte de vitamine D. Ils sont dits non spécifiques lorsque la sélectivité de liaison à l'analyte de vitamine D est faible et qu'ils sont alors capables de se lier à d'autres ligands, tels que d'autres protéines ou anticorps. Selon un mode de réalisation préféré, on préfère les partenaires de liaison spécifiques.

Des anticorps anti-analyte de vitamine D sont connus et notamment décrits dans Hollis, Clin. Chem. 31/11, 1815-1819 (1985) et Hollis, Clin. Chem. 39/3, 529-533 (1993) et dans le brevet EP 1931711. Ils peuvent également être obtenus auprès de diverses Sociétés telles que la Société Bioventix (Angleterre).

Les partenaires de liaison ou les analogues de vitamine D, lorsqu'ils sont utilisés en capture, peuvent être liés ou non à un support, tel que des plaques de micro-titrage, des latex, des cônes de réaction, des billes dont le diamètre est de l'ordre de la centaine de micromètres au nanomètre, par toute technique largement connue de l'homme du métier. Les analogues de vitamine D seront, de préférence, immobilisés sur une phase solide.

Il est possible notamment d'utiliser un support fonctionnalisé avec de l'avidine et/ou de la streptavidine et de venir lier sur cette phase solide un analogue d'analyte biotinylé. Les techniques de fonctionnalisation sont bien connues de l'homme du métier et il pourra y être fait référence.

De manière classique, pour déterminer la quantité de vitamine D et/ou d'au moins un de ses métabolites, le signal, inversement proportionnel à la quantité d'analyte de l'échantillon, pourra être comparé à une courbe de calibration obtenue au préalable par des techniques largement connues de l'homme du métier. Ainsi, par exemple, la courbe de calibration est obtenue en effectuant un dosage immunologique utilisant le même partenaire de liaison, ainsi que des quantités croissantes connues de vitamine D. Une courbe est ainsi obtenue en mettant en abscisse la concentration en vitamine D et en ordonnée le signal correspondant obtenu après dosage immunologique.

Le procédé de détection/quantification selon l'invention peut être directement appliqué au format de tests commerciaux disponibles pour la détection/quantification de la vitamine D. De tels formats de dosage de la vitamine D et/ou d'un de ses métabolites sont notamment commercialisés par Abbott (Architect 25-OH Vitamine D, réf. 3L52), DiaSorin (LIAISON® 25-OH Vitamin D Total assay, réf. 310600), IDS (IDS-iSYS 25-Hydroxy Vitamin D assay, réf. IS-2700), Siemens (ADVIA Centaur® Vitamin D Total, réf. 10491994), Roche (Elecsys Vitamin D total).

La mise en oeuvre d'un immunodosage, de manière classique, inclut donc des réactifs nécessaires à la détection immunologique tels que décrits ci-dessus, lesquels seront incorporés dans l'échantillon. De manière avantageuse, la dissociation, et donc l'incorporation dans l'échantillon à étudier du surfactant fluoroalkyle et de l'alcool en C₁-C₄, de préférence en C₁-C₃, sélectionnés, sera effectuée avant l'incorporation de tels réactifs.

La spectrométrie de masse pourra également être utilisée pour réaliser l'étape de détection/quantification, une fois la dissociation obtenue. Cette technique est une technique d'analyse qui permet la détermination de la masse molaire des composés analysés, ainsi que l'identification de leur structure moléculaire, voire leur quantification. Appliquée à un mélange complexe comme un fluide biologique, elle nécessite d'être couplée à une technique séparative qui permet d'en réduire la complexité. Il s'agit, le plus souvent, de la chromatographie en phase gazeuse (GC) ou de la chromatographie en phase liquide (LC). La spectrométrie de masse en tandem (MS/MS) combine 2 analyseurs et pourra être utilisée aux fins de détection/quantification. Les composés ioniques sélectionnés dans le premier analyseur sont analysés de manière plus fine que dans le second. Cette double analyse permet d'augmenter de manière significative la spécificité de la méthode. Pour cette technologie, on pourra notamment se référer à Van den Broek et al., J. Chromatogr. B 929 161-179 (2013).

L'échantillon biologique avec lequel le procédé de l'invention peut être mis en oeuvre est tout échantillon biologique animal, de préférence humain, susceptible de contenir de l'analyte (vitamine D ou un de ses métabolites), dans lequel un immunodosage ou une analyse en spectrométrie de masse peuvent être mis en oeuvre. Ces échantillons sont largement connus de l'homme du métier. Les échantillons mis en oeuvre dans le procédé de dosage peuvent être préalablement modifiés ou non avant leur utilisation. A titre d'exemple de tels échantillons non préalablement modifiés, on peut citer les fluides biologiques tels que sang total, et à titre d'exemples d'échantillon préalablement modifié, également appelé dérivé d'échantillon, on peut citer le sérum, le plasma, les cellules que l'on récupère à partir d'une biopsie, ou suite à une chirurgie, et que l'on met en culture *in vitro.* Le dosage de la concentration de vitamine D ou d'un de ses métabolites pourra alors être réalisé dans le surnageant de culture, ou encore dans le lysat cellulaire.

Les exemples ci-après permettent d'illustrer l'invention, mais n'ont aucun caractère limitatif.

Les tableaux présentés dans les exemples rapportent pour chaque condition expérimentale testée, le signal de fluorescence (RFV= « relative fluorescence value») déterminé par l'automate VIDAS® (bioMérieux). Souvent, plusieurs mesures indépendantes ont été effectuées pour chacune des conditions. La « moyenne RFV » correspond à la moyenne arithmétique de ces mesures indépendantes.

Pour vérifier l'efficacité des solutions de dissociation, les résultats obtenus avec deux échantillons biologiques ayant des concentrations différentes en 25-OH vitamine D sont comparés, en calculant le rapport en % entre les deux signaux obtenus (noté RFV _{n°2}/ RFV _{n°1}, l'échantillon n°1 étant celui ayant la concentration la plus faible en 25-OH vitamine D). En cas de mesures indépendantes répétées, ce rapport est calculé à partir des moyennes RFV. Plus ce rapport est faible, meilleure est la dissociation.

Le coefficient de variation (CV) est défini comme le rapport entre l'écart-type et la moyenne. Il est souvent exprimé en pourcentage (CV%). Le CV% est une mesure de dispersion relative et reflète la reproductibilité des résultats. Une diminution de la valeur obtenue est signe d'une amélioration de la reproductibilité.

Certaines des expériences présentées font partie de plans d'expériences d'optimisation qui ont été construites en utilisant des tables de Taguchi. Dans le cas où l'optimum est une valeur nominale, comme pour une étude de reproductibilité (valeur de signal fixée), la variance autour de cette valeur peut être considérée comme le résultat des facteurs de bruit, donc comme nuisant à la reproductibilité. A partir des données expérimentales répétées, le ratio signal-sur-bruit (S/B) peut être calculé et est défini selon la formule 10 x log₁₀(moyenne2/ecart-type²). Le ratio S/B, appelée parfois constante de Taguchi, reflète la reproductibilité des résultats. Une augmentation de la valeur obtenue est signe d'une amélioration de la reproductibilité.

Le ratio B/B0% est le signal obtenu pour le point de gamme testé divisé par le signal obtenu pour le point de gamme 0 ng/mL d'analyte, multiplié par 100.

Dans les différents Tableaux, à l'exception du **Tableau 5,** qui vont suivre, les acides perfluoroalkyle utilisés étant liquides, leurs pourcentages sont donnés en volume par rapport au volume total de la solution de dissociation. Pour l'alcool utilisé, dans tous les cas, les pourcentages sont donnés en volume par rapport au volume total de la solution de dissociation.

### EXEMPLE 1 -Intérêt d'une dissociation par un mélange méthanol/acide perfluorohexanoïque par rapport à l'acide perfluorohexanoïque seul

### Préparation des solutions de dissociation

Solution de dissociation comparative : les composants pour préparer le tampon PBS (5 mM de hydrogénophosphate de sodium (Na₂HPO₄), 1,5 mM de dihydrogénophosphate de potassium (KH₂PO₄), 131 mM de NaCl) et 0,75% d'acide perfluorohexanoïque sont dissous dans de l'eau déminéralisée par agitation pendant environ 30 minutes. Le pH est ajusté à 7,2 avec du NaOH 6N.

Solution de l'invention : les composants pour préparer le tampon PBS (5 mM de hydrogénophosphate de sodium (Na₂HPO₄), 1,5 mM de dihydrogénophosphate de potassium (KH₂PO₄), 131 mM de NaCl), 0,75% d'acide perfluorohexanoïque et 5% de méthanol sont dissous dans de l'eau déminéralisée par agitation pendant environ 30 minutes. Le pH est ajusté à 7,2 avec du NaOH 6N.

### Procédé de quantification de la 25-OH vitamine D totale.

Les dosages immunologiques ont été réalisés en utilisant l'automate d'immunoanalyse VIDAS® (bioMérieux). Le cône à usage unique sert à la fois de phase solide pour la réaction et de système de pipetage. La cartouche est composée de 10 puits recouverts d'une feuille d'aluminium scellée et étiquetée. Le premier puits comporte une partie prédécoupée pour faciliter l'introduction de l'échantillon. Le dernier puits est une cuvette optique dans laquelle la fluorescence du substrat est mesurée. Les différents réactifs nécessaires à l'analyse sont contenus dans les puits intermédiaires. Toutes les étapes du test sont réalisées automatiquement par l'instrument Elles sont constituées d'une succession de cycles d'aspiration/refoulement du milieu réactionnel.

### a) Sensibilisation et passivation des cônes

Les cônes ont été sensibilisés avec 300 µL d'une solution d'anticorps anti-protéine porteuse diluée à 10 µg/mL dans un tampon MES 50 mM, pH 6,1. Après 6 h d'incubation à +18/25°C, un lavage est effectué avec une solution de NaCl 9 g/L Ensuite, 300 µL d'une solution de vitamine D couplée à la protéine porteuse, diluée à 150 ng/mL dans un tampon tris 200 mM, pH 6,2 contenant de l'albumine humaine sont ajoutés. La sensibilisation / passivation se poursuit à +18/25°C sur la nuit. Les cônes sont vidés, séchés, puis conservés à +4°C jusqu'à utilisation, à l'abri de l'humidité.

### b) Pré-traitement de l'échantillon

L'échantillon à doser (100 µL) est introduit dans le premier puits de la cartouche. L'échantillon et le réactif de pré-traitement (solution de dissociation comparative ou de l'invention) sont mis en présence pour séparer la vitamine D contenue dans l'échantillon de sa protéine de liaison. L'automate VIDAS® mélange 48 µL de l'échantillon avec 340 µL de la solution de dissociation ; ce mélange est incubé à 37°C pendant 5 minutes.

### c) Mode opératoire de la réaction d'immunodosage

L'échantillon pré-traité (environ 0,9 volume) est transféré dans le puits contenant 1 volume d'un anticorps anti-vitamine D marqué à la phosphatase alcaline (conjugué, bioMérieux). Le conjugué anticorps-phosphatase alcaline est dilué au préalable à environ 10 µg/mL dans un tampon tris 100 mM pH 7,1, NaCl 300 mM contenant de l'albumine humaine. Le mélange échantillon/conjugué est incubé dans le puits pendant environ 5-7 minutes. Puis, le mélange échantillon/conjugué est incubé dans le cône pendant environ 5-7 minutes supplémentaires, durant lesquelles il s'effectue une compétition entre l'antigène présent dans l'échantillon et l'antigène vitamine D fixé sur le cône vis à vis des sites de l'anticorps spécifique anti-vitamine D conjugué. Ensuite, 3 lavages successifs avec un tampon Tris 200 mM pH 8,4, NaCl 300 mM, Tween® 20 0,2% sont effectués afin d'éliminer les composés non fixés. Lors de l'étape finale de révélation, le substrat 4-méthylombelliferyl phosphate est aspiré puis refoulé dans le cône ; l'enzyme du conjugué catalyse la réaction d'hydrolyse de ce substrat en 4-méthylombelliferone dont la fluorescence émise est mesurée à 450 nm. La valeur du signal de fluorescence est inversement proportionnelle à la concentration de l'antigène présent dans l'échantillon.

Le **Tableau 1** ci-dessous récapitule les signaux de fluorescence (RFV) déterminés par l'automate VIDAS® en fonction de la solution de dissociation utilisée, en utilisant 3 échantillons différents de sérum humain. Pour chaque condition expérimentale, 4 mesures indépendantes ont été réalisées.

**Tableau 1 :**

| | Acide perfluorohexanoïque 0,75% SANS méthanol Solution de dissociation comparative | Acide perfluorohexanoïque 0,75% méthanol 5% Solution de l'invention |
|---|---|---|
| | échantillon n°1 à 11 ng/mL | |
| Signal en RFV | 3536 | 3818 |
| | 3457 | 3674 |
| | 3665 | 3889 |
| | 3356 | 3922 |
| moyenne RFV | 3504 | 3826 |
| CV% | 4% | 3% |
| Ratio S/B | 28,58 | 30,82 |
| | | |

| | échantillon n°2 à 20 ng/mL | |
|---|---|---|
| Signal en RFV | 2863 | 3430 |
| | 3290 | 3189 |
| | 3496 3072 | 3316 3149 |
| moyenne RFV | 3180 | 3271 |
| CV% | 9% | 4% |
| Ratio S/B | 21,32 | 28,17 |
| rapport % RFV _{n°2}/RFV_{n°1} | 91% | 85% |
| | | |

| | échantillon n°3 à 36 ng/mL | |
|---|---|---|
| Signal en RFV | 2642 | 2420 |
| | 2440 | 2349 |
| | 2761 | 2490 |
| | 2815 | 2433 |
| moyenne RFV | 2655 | 2423 |
| CV% | 6% | 2% |
| Ratio S/B | 24,1 | 32,43 |
| rapport % RFV _{n°2}/RFV_{n°1} | 76% | 63% |

On constate que l'ajout d'alcool entraîne une amélioration de la dissociation (le rapport de signaux en % diminue), tout en améliorant la reproductibilité (augmentation du ratio signal-sur-bruit et diminution du CV%).

### EXEMPLE 2 - Comparaison de différents alcools

Les solutions de dissociation utilisées dans cet exemple ont été préparées selon le mode opératoire expliqué dans l'exemple 1, dans un tampon PBS à pH 7,2. Les natures et concentrations du surfactant fluoroalkyle et de l'alcool sont variables et sont précisées dans les **Tableaux 2** et **3**.

Pour le reste, on opère comme à l'exemple 1.

**Tableau 2 : Utilisation d'acide perfluorohexanoïque**

| Solution de dissociation comparative : PBS + acide perfluorohexanoïque 1% **sans alcool** | | |
|---|---|---|
| | échantillon n°1 à 11 ng/mL | échantillon n°2 à 35 ng/mL |
| moyenne RFV | 3224 | 2648 |
| rapport % RFV _{n°2}/RFV_{n°3} | - | 82% |
| | | |

| Solution de dissociation de l'invention : PBS + acide perfluorohexanoïque 1% + **méthanol 5%** | | |
|---|---|---|
| | échantillon n°1 à 11 ng/mL | échantillon n°2 à 35 ng/mL |
| moyenne RFV | 3118 | 2323 |
| rapport % RFV _{n°2}/RFV_{n°3} | - | 75% |
| | | |

| Solution de dissociation de l'invention : PBS + acide perfluorohexanoïque 1 % + **éthanol 5%** | | |
|---|---|---|
| | échantillon n°1 à 11 ng/mL | échantillon n°2 à 35 ng/mL |
| moyenne RFV | 2707 | 1925 |
| rapport % RFV _{n°2}/RFV_{n°3} | - | 71% |
| | | |

| Solution de dissociation de l'invention : PBS + acide perfluorohexanoïque 1 % + **isopropanol 5%** | | |
|---|---|---|
| | échantillon n°1 à 11 ng/mL | échantillon n°2 à 35 ng/mL |
| moyenne RFV | 2633 | 2003 |
| rapport % RFV _{n°2}/RFV_{n°1} | - | 76% |

On constate que l'ajout d'alcool, quel qu'il soit, entraîne une amélioration de la dissociation (le rapport de signaux en % diminue).

**Tableau 3 : utilisation d'acide perfluorooctanoïque**

| Solution de dissociation comparative PBS + acide perfluorooctanoïque 0,75% sans alcool | | |
|---|---|---|
| | échantillon n°1 à 11 ng/mL | échantillon n°2 à 35 ng/mL |
| moyenne RFV | 2684 | 1197 |
| rapport % RFV _{n°2}/RFV_{n°3} | - | 45% |
| | | |

| Solution de dissociation : PBS + acide perfluorooctanoïque 0,75% + méthanol 5% | | |
|---|---|---|
| | échantillon n°1 à 11 ng/mL | échantillon n°2 à 35 ng/mL |
| moyenne RFV | 2574 | 787 |
| rapport % RFV _{n°2}/RFV_{n°3} | - | 31 % |
| | | |

| Solution de dissociation : PBS + acide perfluorooctanoïque 0,75% + éthanol 5% | | |
|---|---|---|
| | échantillon n°1 à 11 ng/mL | échantillon n°2 à 35 ng/mL |
| moyenne RFV | 2612 | 759 |
| rapport % RFV _{n°2}/RFV_{n°1} | - | 29% |

On constate que l'ajout d'alcool, quel qu'il soit, entraîne une amélioration de la dissociation (le rapport de signaux en % diminue).

### EXEMPLE 3 - INFLUENCE DE LA CONCENTRATION EN ACIDE

### PERFLUOROALKYLE

Les solutions de dissociations utilisées dans cet exemple ont été préparées selon le mode opératoire expliqué dans l'exemple 1, dans un tampon PBS à pH 7,2.

Pour le reste, on opère comme à l'exemple 1.

**Tableau 4 :**

| | acide perfluorohexanoïque 1,25% sans méthanol | | acide perfluorohexanoïque 1.6% sans méthanol | | acide perfluorohexanoïque 2% sans méthanol | |
|---|---|---|---|---|---|---|
| | échant. n°1 à 15 ng/mL | échant. n°2 à 35 ng/mL | échant. n°1 à 15 ng/mL | échant. n°2 à 35 ng/mL | échant. n°1 à 15 ng/mL | échant. n°2 à 35 ng/mL |
| moyenne RFV | 4724 | 3844 | 4054 | 2885 | 3352 | 2252 |
| rapport % RFV _{n°2}/RFV_{n°1} | - | 81% | - | 71% | - | 67% |
| | | | | | | |

| | acide perfluorohexanoïque 1,25% + méthanol 5% | | acide perfluorohexanoïque 1,6% + méthanol 5% | | acide perfluorohexanoïque 2% + méthanol 5% | |
|---|---|---|---|---|---|---|
| | échant. n°1 à 15 ng/mL | échant. n°2 à 35 ng/mL | échant. n°1 à 15 ng/mL | échant. n°2 à 35 ng/mL | échant. n°1 à 15 ng/mL | échant. n°2 à 35 ng/mL |
| moyenne RFV | 3896 | 2589 | 3245 | 1802 | 2564 | 1335 |
| rapport % RFV _{n°2}/RFV_{n°1} | - | 66% | - | 56% | - | 52% |

Il apparait que la dissociation augmente, en présence d'alcool, dans tous les cas. On constate également qu'en augmentant la concentration en acide perfluoroalkyle, on augmente encore la dissociation.

### EXEMPLE 4 - INFLUENCE DE LA CONCENTRATION EN ALCOOL

Les solutions de dissociation utilisées dans cet exemple ont été préparées selon le mode opératoire expliqué dans l'exemple 1, dans un tampon PBS à pH 7,2. Les natures et concentrations du surfactant fluoroalkyle et de l'alcool sont variables et sont précisées dans le **Tableau 5.** Le perfluorooctanoate d'ammonium étant solide, dans ce cas, les pourcentages sont donnés en masse de perfluorooctanoate d'ammonium par rapport au volume total de la solution.

Pour le reste, on opère comme à l'exemple 1.

**Tableau 5 :**

| | perfluorooctanoate d'ammonium 0,5% sans méthanol | | perfluorooctanoate d'ammonium 0,5% + méthanol 5% | | perfluorooctanoate d'ammonium 0,5% + méthanol 10% | |
|---|---|---|---|---|---|---|
| | échant. n°1 à 15 ng/mL | échant. n°2 à 35 ng/mL | échant. °1 à 15 ng/mL | échant. n°2 à 35 ng/mL | échant. n°1 à 15 ng/mL | échant. n°2 à 35 ng/mL |
| moyenne RFV | 2932 | 2341 | 3944 | 2765 | 4512 | 2697 |
| CV% | 4% | 3% | 4% | 1% | 2% | 3% |
| Ratio S/B | 27,37 | 29,77 | 28,62 | 38,04 | 35,85 | 29,92 |
| rapport % RFV _{n°2}/RFV_{n°1} | - | 80% | - | 70% | - | 60% |

Il apparaît que la dissociation augmente avec la concentration en méthanol, parfois au détriment de la reproductibilité, si cette concentration est trop élevée. La concentration en méthanol sera donc ajustée par l'homme du métier, de manière à trouver un compromis entre la quantité de surfactant perfluoroalkyle et d'alcool.

### EXEMPLE 5 - AJOUT DE PLURONIC® F127

La solution de dissociation de l'inventton utilisée dans cet exemple a été préparée selon le mode opératoire expliqué dans l'exemple 1, dans un tampon Tris 50 mM à pH 7,5, à ceci près que le surfactant fluoroalkyle est l'acide perfluorohéxanoïque à 1,5% et l'alcool est le méthanol à 5%. Le Pluronic® F-127 a été utilisé à une concentration de 0,25%, ou il n'a pas été utilisé. Les résultats sont donnés dans le **Tableau 6** ci-dessous.

Pour le reste, on opère comme à l'exemple 1.

**Tableau 6 :**

| | Sans Pluronic F-127 | Pluronic F-1 27 0,25% |
|---|---|---|
| | moyenne RFV CV% | moyenne RFV CV% |
| point 0 ng/mL | 5388 1% | 5155,5 0% |
| point 11 ng/mL | 3593 5% | 3722 2% |
| point 19 ng/mL | 2693 3% | 3063 2% |
| point 30 ng/mL | 1492 3% | 2024 0% |
| point 102 ng/mL | 295 6% | 413 2% |
| | | |
| échantillon n°1 | 1069 3% | 1597 2% |
| échantillon n°2 | 2179 4% | 2914 2% |
| échantillon n°3 | 188 1 % | 401 1% |
| échantillon n°4 | 625 6% | 1328 2% |

L'ajout d'un surfactant additionnel tel que le Pluronic® F127 permet d'améliorer la reproductibilité (diminution du CV%). L'amélioration de la reproductibilité est plus importante pour les échantillons à forte concentration en Vitamine D.

### EXEMPLE 6 - INFLUENCE DU TAMPON UTILISE

Les solutions de dissociation comparative et de l'invention portant l'indication PBS, utilisées dans cet exemple, ont été préparées selon le mode opératoire expliqué dans l'exemple 1, dans un tampon PBS à pH 7,2. Les natures et les concentrations du surfactant fluoroalkyle et le cas échéant de l'alcool sont indiquées dans les **Tableaux 7** (solution comparative) et **9** (solution de l'invention).

Les solutions de dissociation comparative et de l'invention portant l'indication Tris contiennent 50 mM de Tris, le surfactant fluoroalkyle, avec ou sans alcool. Ces composants sont dissous dans de l'eau déminéralisée par agitation pendant environ 30 minutes. Le pH est ajusté à 7,5 avec du NaOH 6N. Les natures et les concentrations du surfactant fluoroalkyle et le cas échéant de l'alcool sont indiquées dans les **Tableaux 8** (solution comparative) et **9** (solution de l'invention).

**Tableau 7 :**

| Solution de dissociation comparative : PBS + acide perfluoro-hexanoïque à 1,5% | | |
|---|---|---|
| | échantillon n°1 à 11 ng/mL | échantillon n°2 à 30 ng/mL |
| moyenne RFV | 2510 | 1509 |
| rapport % RFV _{n°2}RFV_{n°1} | - | 60% |

**Tableau 8 :**

| Solution de dissociation comparative : Tris + acide perfluorohexanoïque 1,5% | | |
|---|---|---|
| | échantillon n°1 à 11 ng/mL | échantillon n°2 à 30 ng/mL |
| moyenne RFV | 2779 | 1618 |
| rapport % RFV _{n°2}/RFV_{n°1} | - | 58% |

**Tableau 9 :**

| Solution de dissociation de l'invention : tampon PBS bu tampon Tris + acide perfluorohexanoïque 1,5% + méthanol 5% | | | | |
|---|---|---|---|---|
| | échantillon n°1 à 11 ng/mL | | échantillon n°2 à 30 ng/mL | |
| Tampon | PBS | RIS | PBS | TRIS |
| moyenne RFV | 4181 | 4874 | 1665 | 2030 |
| rapport % RFV _{n°2}/RFV_{n°1} | - | - | 40 % | 42% |

Il apparaît que la nature du tampon n'a pas d'influence significative sur le rapport de RFV, et donc sur la dissociation obtenue.

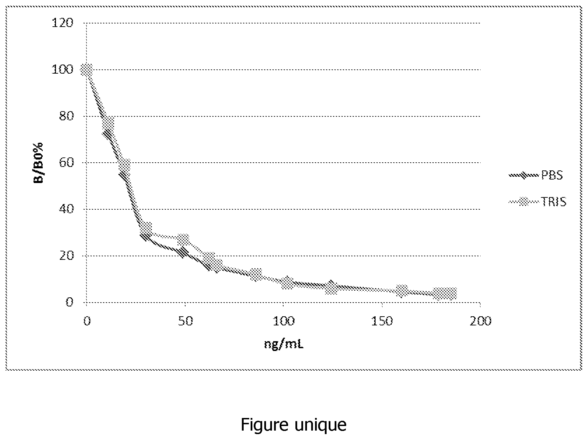

La **Figure unique** présente le ratio B/B0% obtenue sur une gamme de ng/mL d'analyte dans les deux cas (tampon PBS et tampon TRIS). Le ratio B/B0% est le signal obtenu pour le point de gamme testé divisé par le signal obtenu pour le point de gamme 0 ng/mL d'analyte, multiplié par 100.

Il apparaît que le choix du tampon utilisé n'a aucune influence significative sur les résultats obtenus, que ce soit en termes de dissociation ou de reproductibilité.

## Revendications

1. - Utilisation d'au moins un surfactant fluoroalkyle et d'au moins un alcool comportant de 1 à 4 atomes de carbone, pour dissocier, de la protéine de liaison de la vitamine D, la vitamine D et/ou un métabolite de la vitamine D.

2. - Utilisation selon la revendication 1 **caractérisée en ce que** le surfactant fluoroalkyle et l'alcool sont utilisés en quantités telles que le rapport multiplié par 100 de la masse de surfactant, quand ce dernier est un solide, ou du volume de surfactant, quand ce dernier est un liquide, sur le volume d'alcool, appartient à la gamme allant de 10 à 60%, de préférence à la gamme allant de 15 à 40 %, et préférentiellement dans la gamme allant de 15 à 30%.

3. - Utilisation selon la revendication 1 ou 2 **caractérisée en ce que** le surfactant fluoroalkyle est choisi parmi les acides perfluorocarboxyliques, les acides perfluorosulfoniques et leurs sels, et notamment parmi l'acide perfluorohexanoïque, l'acide perfluoroheptanoïque, l'acide perfluorooctanoïque et leurs sels.

4. - Utilisation selon l'une quelconque des revendications 1 à 3 **caractérisée en ce que** l'alcool comporte de 1 à 3 atomes de carbone et est choisi parmi le méthanol, l'éthanol, le *n*-propanol et l'*iso*-propanol.

5. - Utilisation selon l'une quelconque des revendications 1 à 4 **caractérisée en ce que** l'on utilise l'acide perfluorohexanoïque avec le méthanol.

6. - Utilisation selon l'une quelconque des revendications 1 à 5 **caractérisée en ce que** la dissociation est réalisée en présence d'un surfactant additionnel choisi parmi les copolymères blocs à base d'oxyde d'éthylène et d'oxyde de propylène, les polysorbates et les éthers de polyéthylène glycol.

7. - Utilisation selon l'une quelconque des revendications 1 à 6 pour la dissociation, de la protéine de liaison de la vitamine D, de la 25-hydroxyvitamine D, et en particulier de la 25-hydroxyvitamine D₂ et/ou de la 25-hydroxyvitamine D₃.

8. - Utilisation selon l'une des revendications 1 à 7 **caractérisée en ce que** la dissociation est réalisée avec une solution comprenant ledit au moins un surfactant fluoroalkyle et ledit au moins un alcool comportant de 1 à 4 atomes de carbone.

9. - Utilisation selon la revendication 8 **caractérisée en ce que** ladite solution comprend un pourcentage de surfactant fluoroalkyle, en volume lorsque ce dernier est liquide ou en masse lorsque ce dernier est solide, par rapport au volume total de la solution, de 0,1 à 3%, de préférence de 1 à 2%, et/ou un pourcentage en volume d'alcool, par rapport au volume total de la solution, de 0,5 à 10%, de préférence de 2 à 7%.

10. - Utilisation selon la revendication 8 ou 9 **caractérisée en ce que** le surfactant fluoroalkyle et l'alcool sont présents, dans ladite solution, en quantités telles que le rapport multiplié par 100 de la masse de surfactant, quand ce dernier est un solide, ou du volume de surfactant, quand ce dernier est un liquide, sur le volume d'alcool, appartient à la gamme allant de 10 à 60%, de préférence à la gamme allant de 15 à 40 %, et préférentiellement dans la gamme allant de 15 à 30%.

11. - Utilisation selon l'une quelconque des revendications 8 à 10 **caractérisée en ce que** ladite solution contient, en outre, un surfactant additionnel choisi parmi les copolymères blocs à base d'oxyde d'éthylène et d'oxyde de propylène, les polysorbates et les éthers de polyéthylène glycol.

12. - Utilisation selon l'une quelconque des revendications 8 à 11 **caractérisée en ce que** ladite solution est une solution tampon.

13. - Utilisation selon l'une quelconque des revendications 8 à 12 **caractérisée en ce que** ladite solution est tamponnée à un pH appartenant à la gamme allant de 6 à 8.

14. - Utilisation selon l'une quelconque des revendications 8 à 13 **caractérisée en ce que** ladite solution ne contient aucun des composés suivants : diméthyl sulfoxyde, diméthylformamide, N,N-diméthylacétamide, tetraméthylurée, N-méthylpyrrolidone, 1,3-diméthyl-3,4,5,6-tétrahydro-2(1H)-pyrimidone, acide triamide hexaméthylphosphorique.

15. - Procédé de détection et de quantification, *in vitro,* de la vitamine D et/ou d'au moins un métabolite de la vitamine D, dans un échantillon biologique, comprenant les étapes suivantes :
a) une étape de traitement de l'échantillon par incorporation d'au moins un surfactant fluoroalkyle et d'au moins un alcool comportant de 1 à 4 atomes de carbone, de manière à dissocier, la vitamine D et/ou son ou ses métabolite(s) à détecter, de la protéine de liaison de la vitamine D,
b) une étape de détection et de quantification de la vitamine D et/ou d'au moins un de ses métabolites.

16. - Procédé de détection et de quantification, selon la revendication 15 **caractérisé en ce que** le traitement de l'étape a) est réalisé par mélange de l'échantillon avec une solution de dissociation telle que définie à l'une quelconque des revendications 8 à 14.

17. - Procédé de détection et de quantification selon la revendication 16 **caractérisé en ce que** de 1 à 20 volumes de solution de dissociation, de préférence de 5 à 10 volumes, de préférence encore de 6 à 8 volumes, sont utilisés pour 1 volume d'échantillon.

18. - Procédé de détection et de quantification, selon la revendication 16 ou 17 **caractérisé en ce que** l'échantillon biologique est un échantillon de sang, de sérum ou de plasma.

19. - Procédé de détection et de quantification, selon l'une quelconque des revendications 16 à 18 **caractérisé en ce qu'**à l'étape b), la 25-hydroxyvitamine D₂ et/ou la 25-hydroxyvitamine D₃, est(sont) détectée(s).

20. - Procédé de détection et de quantification, selon l'une quelconque des revendications 16 à 19 **caractérisé en ce qu'**à l'étape b) la détection et la quantification sont effectuées par mise en oeuvre d'un immunodosage.

21. - Procédé de détection et de quantification, selon l'une quelconque des revendications 16 à 20 **caractérisé en ce qu'**à l'étape b) la détection et la quantification sont effectuées par spectrométrie de masse.

22. - Trousse de détection et de quantification, de la vitamine D et/ou d'au moins un métabolite de la vitamine D, par immunodosage, comprenant une solution comprenant au moins un surfactant fluoroalkyle et au moins un alcool comprenant de 1 à 4 atomes de carbone telle que définie à l'une quelconque des revendications 8 à 14 et un partenaire de liaison à la vitamine D ou à un de ses métabolites.

23. - Trousse de détection et de quantification, selon la revendication 22 **caractérisée en ce que** le partenaire de liaison est un anticorps marqué et la trousse de détection comporte, en outre, une phase solide sur laquelle est lié un haptène analogue à la vitamine D et/ou au(x) métabolite(s) de la vitamine D à détecter, qui est reconnu par l'anticorps marqué.

## Patentansprüche

1. Verwendung mindestens eines oberflächenaktiven Fluoralkylmittels und mindestens eines Alkohols, umfassend 1 bis 4 Kohlenstoffatome, um von dem Vitamin D-Bindungsprotein das Vitamin D und/oder Vitamin D-Metaboliten zu trennen.

2. Verwendung gemäß Anspruch 1, **dadurch gekennzeichnet, dass** das oberflächenaktive Fluoralkylmittel und der Alkohol in derartigen Mengen verwendet werden, dass das mit 100 multiplizierte Verhältnis der Masse des oberflächenaktiven Mittels, wenn dieses letztgenannte ein Feststoff ist, oder des Volumens des oberflächenaktiven Mittels, wenn dieses letztgenannte eine Flüssigkeit ist, zu dem Alkoholvolumen dem Bereich von 10 bis 60 %, vorzugsweise dem Bereich von 15 bis 40 % und besonders bevorzugt dem Bereich von 15 bis 30 % angehört.

3. Verwendung gemäß Anspruch 1 oder 2, **dadurch gekennzeichnet, dass** das oberflächenaktive Fluoralkylmittel unter den Perfluorcarboxylsäuren, den Perfluorsulfonsäuren und ihren Salzen und insbesondere unter der Perfluorhexansäure, der Perfluorheptansäure, der Perfluoroctansäure und ihren Salzen ausgewählt ist.

4. Verwendung gemäß einem der Ansprüche 1 bis 3, **dadurch gekennzeichnet, dass** der Alkohol 1 bis 3 Kohlenstoffatome umfasst und unter dem Methanol, Ethanol, n-Propanol und Iso-Propanol ausgewählt ist.

5. Verwendung gemäß einem der Ansprüche 1 bis 4, **dadurch gekennzeichnet, dass** die Perfluorhexansäure mit dem Methanol verwendet wird.

6. Verwendung gemäß einem der Ansprüche 1 bis 5, **dadurch gekennzeichnet, dass** die Trennung im Beisein eines zusätzlichen oberflächenaktiven Mittels erfolgt, welches unter den Block-Copolymeren auf Basis von Ethylenoxid und Propylenoxid, den Polysorbaten und den Polyethylenglykolethern ausgewählt ist.

7. Verwendung gemäß einem der Ansprüche 1 bis 6 für die Trennung des Vitamin D-Bindungsproteins von dem 25-Hydroxyvitamin D und insbesondere von dem 25-Hydroxyvitamin D₂ und/oder dem 25-Hydroxyvitamin D₃.

8. Verwendung gemäß einem der Ansprüche 1 bis 7, **dadurch gekennzeichnet, dass** die Trennung mit einer Lösung erfolgt, umfassend das mindestens eine oberflächenaktive Fluoralkylmittel und den mindestens einen Alkohol, umfassend 1 bis 4 Kohlenstoffatome.

9. Verwendung gemäß Anspruch 8, **dadurch gekennzeichnet, dass** die Lösung einen Prozentsatz an oberflächenaktivem Fluoralkylmittel, volumenbezogen, wenn dieses letztgenannte flüssig ist, oder massenbezogen, wenn dieses letztgenannte fest ist, in Bezug zum Gesamtvolumen der Lösung von 0,1 bis 3 %, vorzugsweise von 1 bis 2 %, und/oder einen Volumenprozentsatz an Alkohol in Bezug zum Gesamtvolumen der Lösung von 0,5 bis 10 %, vorzugsweise von 2 bis 7 %, umfasst.

10. Verwendung gemäß Anspruch 8 oder 9, **dadurch gekennzeichnet, dass** das oberflächenaktive Fluoralkylmittel und der Alkohol in der Lösung in derartigen Mengen vorhanden sind, dass das mit 100 multiplizierte Verhältnis der Masse an oberflächenaktivem Mittel, wenn dieses letztgenannte ein Feststoff ist, oder des Volumens an oberflächenaktivem Mittel, wenn dieses letztgenannte eine Flüssigkeit ist, dem Bereich von 10 bis 60 %, vorzugsweise dem Bereich von 15 bis 40 % und besonders bevorzugt dem Bereich von 15 bis 30 % angehört.

11. Verwendung gemäß einem der Ansprüche 8 bis 10, **dadurch gekennzeichnet, dass** die Lösung ferner ein zusätzliches oberflächenaktives Mittel enthält, das unter den Block-Copolymeren auf Basis von Ethylenoxid und Propylenoxid, den Polysorbaten und den Polyethylenglykolethern ausgewählt ist.

12. Verwendung gemäß einem der Ansprüche 8 bis 11, **dadurch gekennzeichnet, dass** die Lösung eine Pufferlösung ist.

13. Verwendung gemäß einem der Ansprüche 8 bis 12, **dadurch gekennzeichnet, dass** die Lösung auf einen pH-Wert, der dem Bereich von 6 bis 8 angehört, gepuffert ist.

14. Verwendung gemäß einem der Ansprüche 8 bis 13, **dadurch gekennzeichnet, dass** die Lösung keine der folgenden Verbindungen enthält: Dimethylsulfoxid, Dimethylformamid, N,N-Dimethylacetamid, Tetramethylharnstoff, N-Methylpyrrolidon, 1,3-Dimethyl-3,4,5,6-tetrahydro-2(1H)-pyrimidon, Hexamethylphosphorsäuretriamid.

15. Verfahren zum Nachweis und zur Quantifizierung des Vitamins D und/oder mindestens eines Metaboliten des Vitamins D in vitro in einer biologischen Probe, umfassend die folgenden Schritte:
a) einen Schritt der Bearbeitung der Probe durch Eingliederung mindestens eines oberflächenaktiven Fluoralkylmittels und mindestens eines Alkohols, umfassend 1 bis 4 Kohlenstoffatome, um das Vitamin D und/oder seinen oder seine zu erkennenden Metaboliten von dem Vitamin D-Bindungsprotein zu trennen,
b) einen Schritt des Nachweises und der Quantifizierung des Vitamins D und/oder mindestens eines seiner Metaboliten.

16. Verfahren zum Nachweis und zur Quantifizierung gemäß Anspruch 15, **dadurch gekennzeichnet, dass** die Behandlung des Schrittes a) durch Mischen der Probe mit einer Trennungslösung, wie in einem der Ansprüche 8 bis 14 definiert, erfolgt.

17. Verfahren zum Nachweis und zur Quantifizierung gemäß Anspruch 16, **dadurch gekennzeichnet, dass** 1 bis 20 Volumina einer Trennungslösung, vorzugsweise 5 bis 10 Volumina, noch bevorzugter 6 bis 8 Volumina, für 1 Volumen Probe verwendet werden.

18. Verfahren zum Nachweis und zur Quantifizierung gemäß Anspruch 16 oder 17, **dadurch gekennzeichnet, dass** die biologische Probe eine Blut-, Serum- oder Plasmaprobe ist.

19. Verfahren zum Nachweis und zur Quantifizierung gemäß einem der Ansprüche 16 bis 18, **dadurch gekennzeichnet, dass** in Schritt b) das 25-Hydroxyvitamin D₂ und/oder das 25-Hydroxyvitamin D₃ erkannt wird(werden).

20. Verfahren zum Nachweis und zur Quantifizierung gemäß einem der Ansprüche 16 bis 19, **dadurch gekennzeichnet, dass** in Schritt b) der Nachweis und die Quantifizierung durch Einsatz eines Immunoassay-Verfahrens erfolgen.

21. Verfahren zum Nachweis und zur Quantifizierung gemäß einem der Ansprüche 16 bis 20, **dadurch gekennzeichnet, dass** in Schritt b) der Nachweis und die Quantifizierung durch Massenspektrometrie erfolgen.

22. Kit zum Nachweis und zur Quantifizierung des Vitamins D und/oder mindestens eines Metaboliten des Vitamins D durch Immunoassay-Verfahren, umfassend eine Lösung, umfassend mindestens ein oberflächenaktives Fluoralkylmittel und mindestens einen Alkohol, umfassend 1 bis 4 Kohlenstoffatome, wie in einem der Ansprüche 8 bis 14 definiert, und einen Bindungspartner mit dem Vitamin D oder einem seiner Metaboliten.

23. Kit zum Nachweis und zur Quantifizierung gemäß Anspruch 22, **dadurch gekennzeichnet, dass** der Bindungspartner ein markierter Antikörper ist, und der Kit zum Nachweis ferner eine feste Phase umfasst, an die ein Hapten analog zum Vitamin D und/oder zu dem(n) Metaboliten des nachzuweisenden Vitamins D gebunden ist, das von dem markierten Antikörper erkannt wird.

## Claims

1. The use of at least one fluoroalkyl surfactant and of at least one alcohol having 1 to 4 carbon atoms for dissociating vitamin D and/or a vitamin D metabolite from vitamin D binding protein.

2. The use according to claim 1, **characterized in that** the fluoroalkyl surfactant and the alcohol are used in quantities such that the ratio multiplied by 100 of the weight of surfactant, when it is solid, or of the volume of surfactant, when it is liquid, over the volume of alcohol lies in the range 10% to 60%, preferably in the range 15% to 40%, and more preferably in the range 15% to 30%.

3. The use according to claim 1 or claim 2, **characterized in that** the fluoroalkyl surfactant is selected from perfluorocarboxylic acids, perfluorosulfonic acids and their salts, and in particular from perfluorohexanoic acid, perfluoroheptanoic acid, and perfluorooctanoic acid, and their salts.

4. The use according to any one of claims 1 to 3, **characterized in that** the alcohol has 1 to 3 carbon atoms and is selected from methanol, ethanol, n-propanol, and isopropanol.

5. The use according to any one of claims 1 to 4, **characterized in that** perfluorohexanoic acid is used with methanol.

6. The use according to any one of claims 1 to 5, **characterized in that** the dissociation is performed in the presence of an additional surfactant selected from block copolymers based on ethylene oxide and propylene oxide, polysorbates, and polyethylene glycol ethers.

7. The use according to any one of claims 1 to 6 for dissociating 25-hydroxy vitamin D, and in particular 25-hydroxy vitamin D₂ and/or 25-hydroxy vitamin D₃ from vitamin D binding protein.

8. The use according to any one of claims 1 to 7 **characterized in that** to dissociation in carried out with a solution comprising the at least one fluoroalkyl surfactant and the at least one alcohol having 1 to 4 carbon atoms.

9. The use according to claim 8, **characterized in that** the solution comprises a percentage of fluoroalkyl surfactant by volume when the surfactant is liquid, or by weight when it is solid, relative to the total volume of the solution lying in the range 0.1% to 3%, preferably in the range 1% to 2%, and/or a percentage by volume of alcohol relative to the total volume of the solution lying in the range 0.5% to 10%, and preferably in the range 2% to 7%.

10. The use according to claim 8 or 9, **characterized in that** the fluoroalkyl surfactant and the alcohol are present, in the said solution, in quantities such that the ratio multiplied by 100 of the weight of surfactant, when it is solid, or of the volume of surfactant, when it is liquid, over the volume of alcohol lies in the range 10% to 60%, preferably in the range 15% to 40%, and more preferably in the range 15% to 30%.

11. The use according to any one of claims 8 to 10, **characterized in that** the said solution further contains an additional surfactant selected from block copolymers based on ethylene oxide and propylene oxide, polysorbates, and polyethylene glycol ethers.

12. The use according to any one of claims 8 to 11, **characterized in that** the said solution is a buffer solution.

13. The use according to any one of claims 8 to 12, **characterized in that** the said solution is buffered to a pH lying in the range 6 to 8.

14. The use according to any one of claims 8 to 13, **characterized in that** the said solution does not contain any of the following compounds: dimethyl sulfoxide, dimethylformamide, N,N-dimethylacetamide, tetramethylurea, N-methylpyrrolidone,1,3-dimethyl-3,4,5,6-tetrahydro-2(1H) -pyrimidone, triamide hexamethyl phosphoric acid.

15. A detection and quantification method for detecting and quantifying, in vitro, vitamin D and/or at least one vitamin D metabolite in a biological sample, the method comprising the following steps:
a) a step of treating the sample by incorporating at least one fluoroalkyl surfactant and at least one alcohol having 1 to 4 carbon atoms, so as to dissociate the vitamin D and/or its metabolite(s) to be detected from vitamin D binding protein; and
b) a step of detecting and quantifying vitamin D and/or at least one of its metabolites.

16. A detection and quantification method according to claim 15, **characterized in that** the treatment of step a) is performed by mixing the sample with a dissociation solution according to any one of claims 8 to 14.

17. A detection and quantification method according to claim 16, **characterized in that** 1 to 20 volumes of dissociation solution, preferably 5 to 10 volumes, more preferably 6 to 8 volumes are used for 1 volume of sample.

18. A detection and quantification method according to claim 16 or 17, **characterized in that** the biological sample is a sample of blood, of serum, or of plasma.

19. A detection and quantification method according to any one of claims 16 to 18, **characterized in that** in step b), 25-hydroxy vitamin D₂ and/or 25-hydroxy vitamin D₃ is/are detected.

20. A detection and quantification method according to any of claims 16 to 19, **characterized in that** in step b), detection and quantification are performed by an immunoassay.

21. A detection and quantification method according to any one of claims 16 to 20, **characterized in that** in step b), detection and quantification are performed by mass spectrometry.

22. A detection and quantification kit for detecting vitamin D and/or at least one vitamin D metabolite by immunoassay, the kit comprising a solution containing at least one fluoroalkyl surfactant and at least one alcohol having 1 to 4 carbon atoms, as defined in one of claims 8 to 14 and a binding partner for vitamin D or one of its metabolites.

23. A detection and quantification kit according to claim 22, **characterized in that** the binding partner is a marked antibody and the detection kit further comprises a solid phase on which there is bonded a hapten analogous to vitamin D and/or to the vitamin D metabolite(s) for detection, which hapten is recognized by the marked antibody.
